(19) Europäisches Patentamt
European Patent Office
Office européen des brevets

(11) Veröffentlichungsnummer: **0 371 461 A2**

# (12) EUROPÄISCHE PATENTANMELDUNG

(21) Anmeldenummer: **89121940.4**

(51) Int. Cl.5: **C07D 209/48, A61K 31/40**

(22) Anmeldetag: **28.11.89**

Patentansprüche für folgende Vertragsstaaten: ES + GR.

(30) Priorität: **30.11.88 DE 3840354**

(43) Veröffentlichungstag der Anmeldung: **06.06.90 Patentblatt 90/23**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lang, Hans-Jochen, Dr.**
**Rüdesheimer Strasse 7**
**D-6238 Hofheim am Taunus(DE)**
Erfinder: **Granzer, Ernold, Dr. Dr.**
**Falkensteiner Strasse 24**
**D-6233 Kelkheim(Taunus)(DE)**
Erfinder: **Hropot, Max, Dr.**
**Friedrich-Stolz-Strasse 13**
**D-6093 Flörsheim am Main(DE)**
Erfinder: **Kerekjarto, Bela, Dr.**
**Weilbächer Wälder FA8**
**D-6238 Hofheim am Taunus(DE)**

## (54) Benzolsulfonamid-Derivate und Verfahren zu ihrer Herstellung.

(57) Verbindungen I

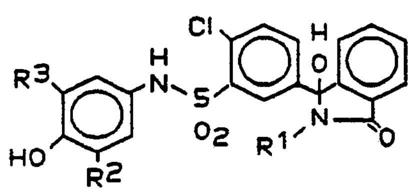

I

haben eine diuretische, saluretische und hypolipidämische Wirkung.
Sie werden aus II

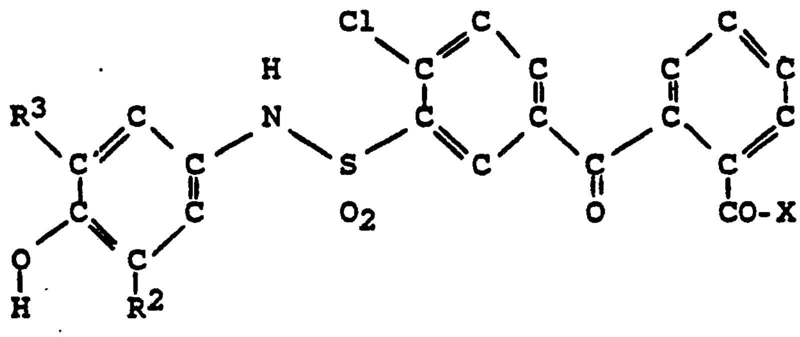

II

durch Umsetzung mit einem Amin III
$R^1 - NH_2$ III

EP 0 371 461 A2

erhalten.

## Benzolsulfonamid-Derivate und Verfahren zu ihrer Herstellung

Es ist bekannt, daß das Benzolsulfonamid-Derivat Chlorthalidon

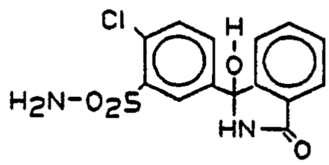

eine starke diuretische und saluretische Wirkung besitzt.

Im Verlauf der langjährigen therapeutischen Verwendung der Verbindung als Diuretikum und Antihypertensivum stellte sich neben anderen arzneimittelbedingten Nebenwirkungen insbesondere eine ungünstige Beeinflussung der Zusammensetzung der Serumlipide heraus. Es ist bekannt, daß derartige Veränderungen zugunsten der atherogenen Lipide der LDL-Fraktion das Risiko einer atherosklerotischen Gefäßschädigung erhöhen.

In einer kürzlich erschienenen Patentpublikation wird die Erfindung von Diuretika mit einer günstigen Wirkung auf die Serumlipidzusammsetzung beschrieben (EP 0 288 028). In dieser Entgegenhaltung findet sich jedoch keinerlei Hinweis auf Verbindungen, die ein Isoindolinonsystem enthalten.

Die Erfindung hat sich die Aufgabe gestellt, Benzolsulfonamidderivate mit weiter verbesserten Eigenschaften zur Verfügung zu stellen.

Dies ist gelungen mit Benzolsulfonamidderivaten der Formel I

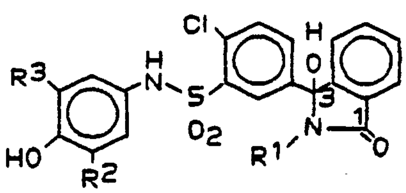

I ,

worin

$R^1$ Wasserstoff oder Methyl

$R^2$ Wasserstoff oder einen Alkylrest mit 1 - 6,

$R^3$ einen Alkylrest mit 3 bis 5 C-Atomen bedeuten,

sowie die entsprechenden zu I offenkettigen tautomeren Formen der Formel Ia

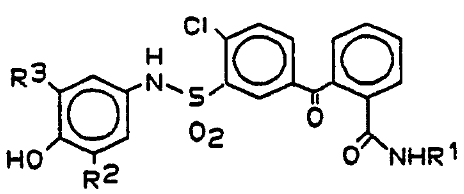

Ia)

worin $R^1$ bis $R^3$ die angegebene Bedeutung besitzen.

Bevorzugt sind solche Verbindungen der Formel I, worin $R^1$ Wasserstoff, $R^2$ Methyl, Isopropyl oder tert. Butyl und $R^3$ Isopropyl oder tert. Butyl bedeuten.

Besonders bevorzugt ist als herausragende Einzelverbindung das Di-tert.butyl-Derivat 3-[4-Chlor-3-(3,5-Di-tert.butyl-4-hydroxyphenylsulfamoyl)-phenyl]-3-hydroxy-1-isoindolinon zu nennen.

Die Erfindung betrifft weiterhin ein Verfahren zur Herstellung der Verbindungen der Formel I, das dadurch gekennzeichnet ist,

daß man Verbindungen der Formel II

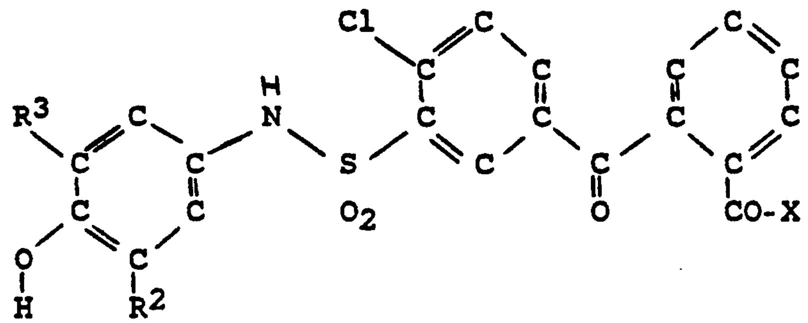

II

,

mit einem Amin der Formel III

$R^1$ - $NH_2$ III

umsetzt, worin $R^1$ bis $R^3$ die angegebene Bedeutung besitzen.

Die Verbindungen I können über eine Tautomerie mit den Verbindungen der Formel Ia im Gleichgewicht stehen : I ⇄ Ia. Welche der beiden Formen I oder Ia einer erfindungsgemäßen Verbindung mit überwiegendem Anteil in dem Gleichgewicht vorliegt, hängt vom Lösungsmittel, der Temperatur und vom Substituenten $R^1$ ab. Die cyclische tautomere Form I ist bevorzugt.

Die erfindungsgemäßen Verbindungen der Formel I können außerdem in ihren möglichen geometrischen isomeren Formen vorliegen.

Die Alkylreste der Substituenten $R^2$ und $R^3$ können sowohl geradkettig als auch verzweigt sein.

Das Verfahren wird vorteilhaft so ausgeführt, daß man beispielsweise Verbindungen der Formel II, worin $R^2$ und $R^3$ die angegebene Bedeutung besitzen und X der O-($C_1$-$C_6$)-Acylrest eines gemischten Anhydrides ist, mit Aminen der Formel III zur Reaktion bringt. Die gemischten Anhydride werden vorteilhaft dadurch in situ erzeugt, daß an Verbindungen der Formel II, worin X für -OM steht und M Wasserstoff bedeutet, mit einem Äquivalent einer geeigneten Base, beispielsweise einem Alkali- oder Erdalkalihydroxid wie KOH, NaOH, $Ca(OH)_2$, einem Alkalimetallcarbonat oder -hydrogencarbonat wie $Na_2CO_3$, $K_2CO_3$, oder mit einem vorzugsweise tertiärem Amin wie Trimethylamin, Triethylamin, Tripropylamin, Tributylamin, Dicyclohexylethylamin, in die entsprechenden Carbonsäuresalze der Formel II überführt, worin X OM bedeutet und M für Na, K, Ca oder eine Trialkylammoniumkation, wie $[HN(Ethyl)_3]^+$ steht, die anschließend mit 1 bis 2,5 Mol, bevorzugt mit 1 bis 1,5 Mol eines aktivierten Säurederivates zu einem gemischten Anhydrid umgesetzt werden.

Man verwendet als aktiviertes Säurederivat vorteilhaft beispielsweise einen Chlorameisensäurealkylester, bevorzugt Chlorameisensäureethylester oder Chlorameisensäuremethylester, ein Carbamidsäurechlorid (wie N,N-Dimethylcarbamidsäurechlorid oder N,N-Diethylcarbamidsäurechlorid) oder das Säurechlorid einer aliphatischen oder aromatischen Sulfonsäure, beispielsweise Methan-, Ethan-, Benzol- oder p-Toluolsulfonsäurechlorid.

Die Reaktion führt man vorteilhaft in einem wasserfreien, polaren, organischen Lösungsmittel, beispielsweise in Aceton, Methylethylketon, einem Alkansäure-($C_1$-$C_6$)-alkylester wie Essigsäuremethyl- oder -ethylester, einem Alkansäureamid, beispielsweise Dimethylformamid, Dimethylacetamid, in Dimethylsulfoxid, in Acetonitril, in einem niederen aliphatischen Alkohol, z. B. in Methanol, Ethanol oder Isopropanol, bevorzugt in einem ringförmigen oder offenkettigen Ether und Polyether, wie Tetrahydrofuran, Dioxan, Ethylenglycoldimethylether, Diethylenglycoldimethylether durch. Die Reaktionsdauer für einen 0,1 molaren Ansatz beträgt 1 Minute bis 5 Stunden, wobei man das Gemisch bevorzugt über einen Zeitraum von 5 bis 60 Minuten rühren läßt. Die Reaktion kann in einem Temperaturbereich von -50° C bis 100° C durchgeführt werden, wobei man vorteilhaft zwischen -30° C und + 30° C, bevorzugt zwischen -10° C und +15° C arbeitet.

Obgleich die gemischten Anhydride gefaßt werden können, indem man beispielsweise das Lösungsmittel bei Temperaturen zwischen -5° C und +10° C verdampft und mit einem geeigneten Solvens, wie Essigsäureethylester, aus dem Rückstand extrahiert, verfährt man vorteilhaft so, daß man auf die Isolierung des gemischten Anhydrides verzichtet und das Amin der Formel III zum Reaktionsgemisch gibt. Das Amin kann sowohl unverdünnt als auch in Form einer Lösung zugegeben werden, wobei man als Lösungsmittel vorteilhaft dasjenige des Reaktionsgemisches oder eines der hierfür angegebenen Solvenzien sowie auch wäßrige Lösungen des Amins oder von Ammoniak verwenden kann. Dabei kommt, bezogen auf die Verbindung der Formel II, mindestens 1 Mol, vielfach vorteilhaft aber ein deutlicher molarer Überschuß an Amin (10-fach und mehr) zur Anwendung. Die Reaktion wird in einem Temperaturbereich zwischen -30° C und +100° C, bevorzugt zwischen +5 ° C und +40 ° C durchgeführt. Die Reaktionszeit liegt zwischen 10

Minuten und 5 Tagen, wobei der Reaktionsfortgang dünnschichtchromatografisch, vorteilhaft an Kieselgel, verfolgt werden kann.

Die aus Verbindungen der Formel II mit X in der Bedeutung von OH mit $COCl_2$, Oxalylchlorid, $POCl_3$, $SoCl_2$, $PCl_3$ oder $PCl_5$ darstellbaren Säurechloride der Formel II, worin X für Chlor steht, werden prinzipiell wie die gemischten Anhydride in der eben beschriebenen Weise umgesetzt.

Eine ebenfalls vorteilhafte Variante der Verfahrensweise a) besteht in der Umsetzung der Carbonsäuren der Formel II mit X in der Bedeutung von OH mit 1 Mol Carbonyldiimidazol, wobei unter den angegebenen milden Reaktionsbedingungen in einem inerten polaren Lösungsmittel das aktivierte Carbonsäureimidazolid der Formel II mit X = 1-Imidazolyl gebildet wird, das unter analogen Bedingungen wie das oben beschriebene gemischte Säureanhydrid mit einem Amin der Formel III in die erfindungsgemäßen Verbindungen der Formel I übergeführt werden kann.

Eine weitere vorteilhafte Variante der Verfahrensvariante a) besteht in der Umsetzung von Verbindungen der Formel II, worin $R_1$ bis $R_3$ die angegebene Bedeutung besitzen und X eine niedere Alkoxygruppe mit 1 bis 6 C-Atomen im Alkylteil, bevorzugt Methoxy und Ethoxy, oder einen ggf. durch F, Cl oder Br substituierten Phenyloxyrest bedeutet, mit einem Amin der Formel III. Die Reaktion führt man vorteilhaft in Wasser oder einem polaren, gegenüber Aminen inerten organischen Lösungsmittel, beispielsweise in einem niederen Alkansäureamid, wie Dimethylformamid, Dimethylacetamid, Dimethylsulfoxid, in einem ringförmigen oder offenkettigen Ether oder Polyether, wie Tetrahydrofuran, Dioxan, Ethylenglycoldimethylether, bevorzugt aber in einem niederen Alkohol, wie Methanol, Ethanol, Propanol, Isopropanol oder in reinem unverdünntem Amin der Formel III ohne Anwendung eines Lösungsmittels aus. Man arbeitet bevorzugt in einem Temperaturbereich 10 bis 60 °C, besonders bevorzugt bei 15 bis 30 °C. Das Ende der Reeaktion wird vorteilhaft dünnschichtchromatografisch an Kieselgel bestimmt. Die Reaktionsdauer beträgt erfahrungsgemäß je nach angewandter Reaktionstemperatur und Aminokomponente zwischen einer Stunde und 14 Tagen, beispielsweise bei Zimmertemperatur zwischen 5 und 72 Stunden. Die Carbonsäureester der Formel II und die Amine der Formel III werden bevorzugt in einem Molverhältnis von 1:1 bis 1:3 umgesetzt, wobei aber das Amin auch in einem bis zu 10fach molaren Überschuß angewendet werden kann. Die Verbindung der Formel II, worin $R^1$ bis $R^3$ die angegebene Bedeutung besitzen und X für $-O-(C_1-C_6)$-Alkyl steht, gewinnt man in an sich bekannter Weise durch die Einwirkung eines niederen Alkanols mit 1 bis 6 C-Atomen, bevorzugt von Methanol, Ethanol, Propanol, Isopropanol und Butanol, z. B. in Gegenwart eines organischen oder anorganischen Säurechlorides, durch Protonenkatalyse.

In gleicher Weise lassen sich ebenfalls die entsprechenden aktivierten Ester der Formel II mit X = CN, $N_3$, $-O-CH_2CN$ mit einem Amin der Formel III zur Reaktion bringen.

Eine weitere vorteilhafte Variante der Verfahrensweise a) besteht in der Umsetzung von Verbindungen der Formel II, worin $R^2$ und $R^3$ die angegebene Bedeutung besitzen und X einen 1-Benztriazoloxy-Rest bedeutet, mit einem Amin der Formel III. Diese aktivierten Carbonsäure-1-benztriazoloxy-ester erhält man in an sich bekannter Weise durch Umsetzung der Carbonsäuren der Formel II mit X in der Bedeutung von OH mit 1-Hydroxybenztriazol in Gegenwart von Dicyclohexylcabodiimid, wobei vorteilhaft in einem polaren organischen Lösungsmittel, wie in Dimethylformamid, Dimethylacetamid, zwischen 0 ° und 60 °C gearbeitet wird.

Die Herstellung der Carbonsäuren der Formel II mit X in der Bedeutung von OH gewinnt man durch Umsetzung des literaturbekannten Sulfochlorids der Formel IV mit einem Anilin-Derivat der Formel V,

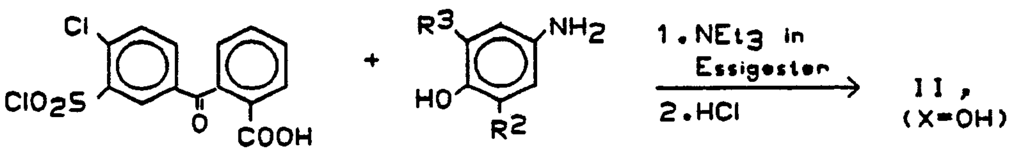


IV V

worin $R^2$ und $R^3$ die angegebene Bedeutung besitzen, in Gegenwart einer geeigneten anorganischen oder organischen Base.

Als Basen verwendet man vorteilhaft Alkalicarbonate oder -hydrogencarbonate oder organische Basen, wie Triethylamin, Pyridin, Dimethylaminopyridin. Man arbeitet vorteilhaft in einem polaren protischen oder aprotischen Lösungsmittel, beispielsweise in Methanol, Ethanol, Isopropanol, Essigsäureethylester, Tetrahydrofuran, Dimethoxyethan, Dimethylsulfoxid, Dimethylformamid, Dimethylacetamid, in einem bevorzugten Temperaturbereich zwischen 20 ° und 80 °C. Die bevorzugte Aufarbeitung nach der Reaktion erfolgt durch teilweises oder vollständiges Verdampfen des Lösungsmittels unter vermindertem Druck und nachfolgendem Versetzen mit Salzsäure oder Schwefelsäure, was zur Abscheidung der Carbonsäure führt.

Erfindungsgemäß können außer den in den Ausführungsbeispielen beschriebenen Benzolsulfonamiden beispielsweis auch die folgenden Verbindungen der Struktur I dargestellt werden:
3-[4-Chlor-3-(3,5-diisopropyl-4-hydroxyphenyl-sulfamoyl)-phenyl]-3-hydroxy-1-isoindolinon,
3-[4-Chlor-3-(3-tert.butyl-5-methyl-4-hydroxyphenyl-sulfamoyl)-phenyl]-3-hydroxy-1-isoindolinon.

Die Verfahrensprodukte sind wertvolle Arzneimittel und zeichnen sich durch eine Vielzahl sehr günstiger therapeutisch verwertbarer Eigenschaften, insbesondere durch die Kombination ihrer sehr guten diuretischen und saluretischen Wirkungen mit ausgeprägten antiatherosklerotischen Eigenschaften aus.

Dies ist insbesondere deshalb von erheblichem therapeutischen Interesse, weil die bislang im Handel befindlichen Diusaluretika diejenigen Lipidanteile (VLDL-und LDL-Fraktionen) des Blutes, die für ein Auslösen oder Fortschreiten der Atherosklerose verantwortlich gemacht werden, entweder nicht vermindern oder sogar vermehren, so daß insbesondere im letzten Fall derartige Wirkstoffe ein gewisses atherogenes Potential besitzen.

Diuretika gehören auch noch heute wegen ihrer zahlreichen günstigen Eigenschaften und ihrer relativ guten Verträglichkeit zu den Basistherapeutika in der Bluthochdruckbehandlung.
Da der Altersbluthochdruck zu einem wesentlichen Anteil Folge und Initiator atherosklerotischer Gefäßveränderungen ist, die wiederum den Bluthochdruck verstärken, sind diusaluretische Wirkstoffe mit einer Verminderung des atherosklerotischen Risikos durch Senkung insbesondere der atherogenen LDL-Fraktion des Cholesterins im Blut von besonderer therapeutischer Bedeutung.

Es war überraschend, daß die Verbindungen der Formel I, die sich vom Chlorthalidon durch Einführung eines substituierten Phenylrestes an der Sulfonamidgruppe unterscheiden, neben der diuretischen und saluretischen Wirkung eine den Serumspiegel der atherogenen Lipidfraktionen senkende Wirkung besitzen. Ein solches Wirkprofil bedeutet einen therapeutischen Fortschritt in der Behandlung der Hypertonie des älteren Menschen.

Aus zahlreichen Publikationen der letzten Jahre geht hervor, daß der Entstehung atherosklerotischer Plaques Verletzungen des Gefäßendothels vorausgehen. Derartige Endothelverletzungen können durch Fettsäureperoxide verursacht werden, so daß mit einer Verminderung dieser Lipidperoxide, die im wesentlichen in der LDL-Fraktion transportiert werden, zwangsläufig eine Verminderung des atherogenen Risikos verbunden ist. Die erfindungsgemäßen Verbindungen vermögen die Bildung von Malondialdehyd, der ein Maß für die Lipidperoxidation ist, zu unterdrücken. Die antioxidative Wirkkomponente läßt sich auf den Hydroxyphenylamino-Substituenten an der Sulfonylgruppe der Verbindungen I zurückführen.

Die erfindungsgemäßen Verbindungen vermindern nicht nur das Fortschreiten der Atherosklerose durch Reduktion der atherogenen Cholesterinfraktion und durch die Reduktion des erhöhten Blutdrucks, sondern wirken darüber hinaus durch die Zerstörung der Lipidperoxide präventiv einer Neubildung atherosklerotischer Plaques entgegen.

Die erfindungsgemäßen Verbindungen I zeigen im Tierversuch (Ratte) eine mit handelsüblichen Diuretika, wie Hydrochlorothiazid und Chlorthalidon, vergleichbare salidiuretische Wirkung. Darüber hinaus zeichnen sich die Verbindungen I durch eine langanhaltende Wirkungsdauer aus. Deshalb sind die erfindungsgemäßen Verbindungen I auch infolge ihrer diusaluretischen Wirkung zur Behandlung hypertoner Zustände geeignet, wobei man sie, wie heute allgemein üblich, gegebenenfalls mit einem anderen Antihypertonikum kombinieren wird.

Die Verbindungen I werden in Dosen von mindestens 0,5 mg/kg und Tag, bevorzugt 1 mg/kg und Tag bis höchstens 10 mg/kg/Tag, vorzugsweise bis 5 mg/kg/Tag angewendet, bezogen auf einen erwachsenen Menschen von etwa 75 kg Körpergewicht.

Als therapeutische Zubereitung der neuen Verbindungen kommen vor allem Tabletten, Dragees, Kapseln und Suppositorien sowie auch Ampullen zur parenteralen Verabreichung (i.v., s.c. und i.m.) in Frage. Die therapeutische Einheitsdosis liegt zwischen 0,5 und 500 mg, vorzugsweise zwischen 10 und 300 mg pro Tablette.

Diese Zubereitungen können speziell bei der Behandlung des Bluthochdrucks außer den üblichen Füll- und Trägerstoffen noch ein Antihypertensivum, wie beispielsweise Reserpin, Hydralazin, Guanethidin, $\alpha$-Methyldopa, Clonidin, einen ß-sympathikolytischen Wirkstoff, wie Propranolol, oder einen ACE-Inhibitor, wie Captopril, Enalapril oder Ramipril enthalten.

Außerdem sind therapeutische Kombinationspräparate mit kaliumretinierenden Verbindungen, wie Aldosteronantagonisten, z. B. Spironolacton oder Pseudoaldosteronantagonisten, wie Triamteren oder Amilorid, von Interesse. Weiterhin kommt Kalium-Substitution in verschiedenen Anwendungsformen, z. B. Dragees, Tabletten, Brausetabletten, Säfte u. a. in Frage.

Von therapeutischem Interesse können ebenfalls Kombinationen der erfindungsgemäßen Verbindungen mit einem antihyperurikämisch und/oder urikosurisch wirksamen Mittel sein, das entweder durch eine

Hemmung der Xanthinoxidase oder durch Erhöhung der renalen Harnsäureausscheidung stärkere Blutharnsäureerhöhungen vermeidet.

Im nachfolgenden Beispiel sind Schmelz- und Zersetzungspunkt nicht korrigiert.

**Beispiel 1:**

**3-[4-Chlor-3-(3,5-di-tert.butyl-4-hydroxyphenyl-sulfamoyl)-phenyl]-3-hydroxy-1-isoindolinon**

a) 4′-Chlor-3′-(3,5-di-tert.butyl-4-hydroxyphenyl-sulfamoyl)-benzophenon-2-carbonsäure

Zu einer Lösung von 14,3 g 4′-Chlor-3′-chlorsulfonyl-benzophenon-2-carbonsäure in 70 ml Ethylacetat gibt man eine Mischung aus 8,6 g 4-Amino-2,6-di-tert.butyl-phenol, 80 ml Essigester und 6 ml Triethylamin. Nach Abklingen der exothermen Reaktion erwärmt man 2 Stunden auf 55° C, versetzt sodann bei Zimmertemperatur mit 300 ml Wasser und stellt die wäßrige Phase mit 2 N Salzsäure auf pH 1. Man trennt die organische Phase ab, wäscht diese einmal mit Wasser. Nach Trocknen über Magnesiumsulfat destilliert man das Lösungsmittel ab und bringt den viskosen Rückstand nach Zugabe von 50 ml Isopropanol und 250 ml n-Hexan zur Kristallisation. Nach mehrtägigem Stehenlassen filtriert man die Kristalle ab und löst mehrfach aus Isopropanol/n-Hexan um.
Farblose bis blaß rötlich gefärbte Kristalle,
Schmp. 150° - 152° C.

b) 3-[4-Chlor-(3,5-di-tert.butyl-4-hydroxyphenyl-sulfamoyl)-phenyl]-3-hydroxy-1-isoindolinon

Zu einer Lösung aus 8,7 g 4′-Chlor-3′-(3,5-di-tert.butyl-4-hydroxyphenyl-sulfamoyl)-benzophenon-2-carbonsäure und 2,38 g 1-Hydroxybenztriazol in 50 ml wasserfreiem Dimethylformamid tropft man unter Ausschluß von Luftfeuchtigkeit unter magnetischer Rührung bei Zimmertemperatur eine Lösung von 3,3 g Dicyclohexylcarbodiimid in 50 ml wasserfreiem Dimethylformamid. Man rührt nach Abklingen der schwach exothermen Reaktion 3 Stunden bei Zimmertemperatur und filtriert vom Dicyclohexylharnstoff-Niederschlag ab. Nach Zutropfen in eine 25 %ige wäßrige Ammoniaklösung wird 30 Min. bei Zimmertemperatur nachgerührt, mit 2 N HCl auf pH 1 - 2 gestellt und mit Essigester extrahiert. Man trocknet die organische phase über Magnesiumsulfat, destilliert das Lösungsmittel ab, bringt den amorphen Rückstand unter 50 ml warmem Diisopropylether zur Kristallisation und reinigt das Produkt durch Säulenchromatographie an 150 g Kieselgel (0,063 - 0,200) mit Essigester/Toluol = 1 : 3 bis 1 : 1.
Farblose Kristalle, Fp. 145° - 148° C (Zers.).

**Ansprüche**

1. Verbindung der Formel I

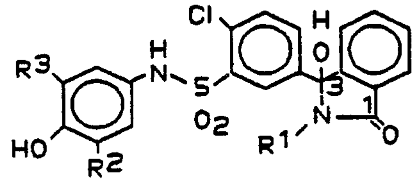

I ,

worin
$R^1$ Wasserstoff oder Methyl
$R^2$ Wasserstoff oder einen Alkylrest mit 1 - 6,
$R^3$ einen Alkylrest mit 3 bis 5 C-Atomen bedeuten,
sowie die entsprechenden zu I offenkettigen tautomeren Formen der Formel Ia

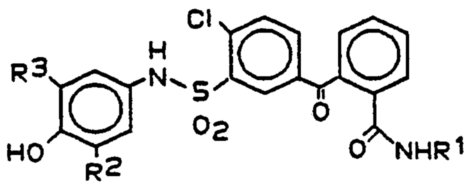

Ia),

worin $R^1$ bis $R^3$ die angegebene Bedeutung besitzen.

2. Verbindung I nach Anspruch 1, dadurch gekennzeichnet, daß $R^1$ Wasserstoff, $R^2$ Methyl, Isopropyl oder tert.Butyl und $R^3$ Isopropyl oder tert.Butyl bedeuten.

3. Verbindung nach Anspruch 1, dadurch gekennzeichnet, daß sie 3-[4-Chlor-3-(3,5-Di-tert.butyl-4-hydroxyphenyl-sulfamoyl)phenyl]-3-hydroxy-1-isoindolinon ist.

4. Verfahren zur Herstellung einer Verbindung der Formel I nach Anspruch 1

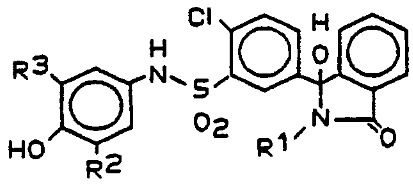

I,

worin
$R^1$ Wasserstoff oder Methyl
$R^2$ Wasserstoff oder einen Alkylrest mit 1 - 6,
$R^3$ einen Alkylrest mit 3 bis 5 C-Atomen bedeuten,
sowie der entsprechenden zu I offenkettigen tautomeren Formen der Formel Ia

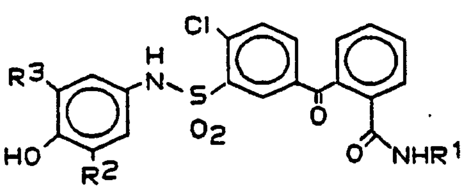

Ia)

worin $R^1$ bis $R^3$ die angegebene Bedeutung besitzen,
dadurch gekennzeichnet, daß man Verbindungen der Formel II

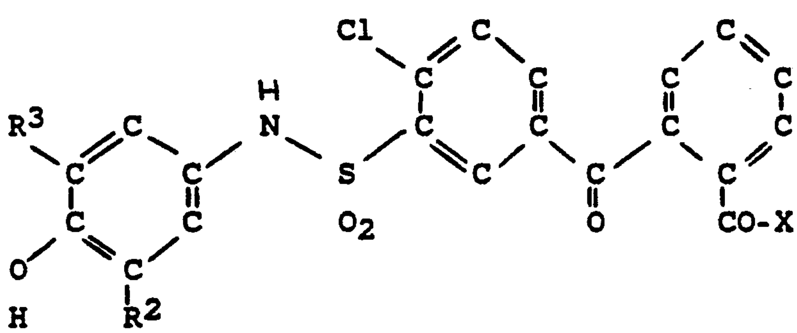

II

mit einem Amin der Formel III
$R^1 - NH_2$ III
umsetzt, worin $R^1$ bis $R^3$ die angegebene Bedeutung besitzen und gegebenenfalls in das Salz überführt.

5. Verbindung I nach Anspruch 1 zur Anwendung als Blutfett-senkendes Heilmittel.

6. Verwendung einer Verbindung I nach Anspruch 1 zur Herstellung eines Medikaments mit Blutfett-

senkender Wirkung.

Patentansprüche für folgende Vertragsstaaten : ES, GR

1. Verfahren zur Herstellung einer Verbindung der Formel I

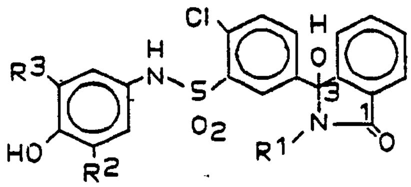

I ,

worin
$R^1$ Wasserstoff oder Methyl
$R^2$ Wasserstoff oder einen Alkylrest mit 1 - 6,
$R^3$ einen Alkylrest mit 3 bis 5 C-Atomen bedeuten,
sowie der entsprechenden zu I offenkettigen tautomeren Formen der Formel Ia

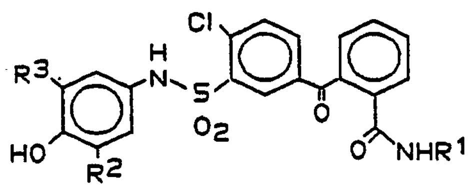

Ia) ,

worin $R^1$ bis $R^3$ die angegebene Bedeutung besitzen, dadurch gekennzeichnet, daß man Verbindungen der Formel II

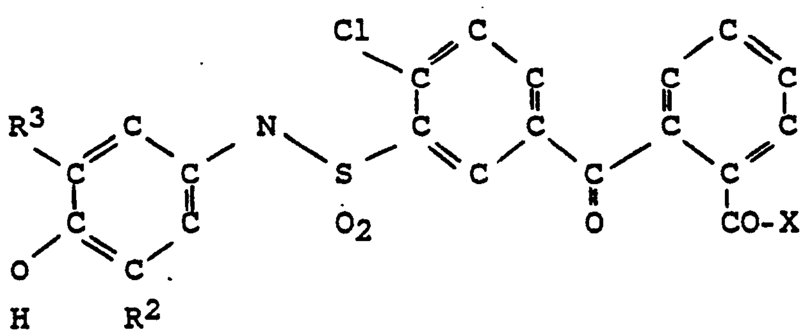

II

mit einem Amin der Formel III
$R^1 - NH_2$ III
umsetzt, worin $R^1$ bis $R^3$ die angegebene Bedeutung besitzen und gegebenenfalls in das Salz überführt.

2. Verfahren nach Anspruch 1, dadurch gekennzeichnet, daß $R^1$ Wasserstoff, $R^2$ Methyl, Isopropyl oder tert.Butyl und $R^3$ Isopropyl oder tert.Butyl bedeuten.

3. Verfahren nach Anspruch 1, dadurch gekennzeichnet, daß 3-[4-Chlor-3-(3,5-Di-tert.butyl-4-hydroxyphenyl-sulfamoyl)phenyl]-3-hydroxy-1-isoindolon dargestellt wird.

4. Verfahren zum Herstellen eines Medikaments mit Blutfett-senkender Wirkung, dadurch gekennzeichnet, daß man eine wirksame Menge einer Verbindung I nach Anspruch 1 mit den üblichen Zuschlagstoffen versieht.